# EUROPÄISCHE PATENTSCHRIFT

(11) **EP 2 900 187 B1**
(45) Veröffentlichungstag und Bekanntmachung des Hinweises auf die Patenterteilung: **04.03.2020**
(21) Anmeldenummer: 13789167.7
(22) Anmeldetag: 20.09.2013
(51) Int. Cl.: A61F 9/007

(54) **STEUERUNGSVORRICHTUNG FÜR EIN OPHTHALMOCHIRURGISCHES SYSTEM**
CONTROL DEVICE FOR AN OPHTHALMIC SURGICAL SYSTEM
DISPOSITIF DE COMMANDE POUR UN SYSTÈME OPHTALMO-CHIRURGICAL

(30) Priorität: 27.09.2012 DE 102012018982
(43) Veröffentlichungstag der Anmeldung: 05.08.2015
(73) Patentinhaber: Carl Zeiss Meditec AG, 07745 Jena (DE)
(72) Erfinder: EICHLER, Michael, 73434 Aalen (DE)
(74) Vertreter: Carl Zeiss AG - Patentabteilung
(86) Internationale Anmeldenummer: PCT/EP2013/002835
(87) Internationale Veröffentlichungsnummer: WO 2014/048552

(56) Entgegenhaltungen:
- WO-A1-2007/073802
- WO-A1-2012/041289
- DE-A1- 19 718 139

## Beschreibung

Die Erfindung betrifft eine Steuerungsvorrichtung für ein ophthalmochirurgisches System zur Phakoemulsifikation einer Augenlinse und ein ophthalmochirurgisches System mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Nadel in die Augenlinse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Partikel und des Fluides durch eine Aspirationsleitung erfolgt, welche üblicherweise innerhalb der Nadel angeordnet ist. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Eine Operation des Grauen Stars bzw. eine Kataraktoperation ist ein Eingriff mit einer relativ geringen Komplikationsrate und großer Patientenzahl. Die relativ geringe Komplikationsrate lässt sich jedoch nur erreichen, wenn ein Operateur mit viel Erfahrung die Operation durchführt. Bei der Zerkleinerung der Augenlinse durch eine mit Ultraschall schwingende Nadelspitze ist es unvermeidbar, dass sich während der Operation ein relativ großes Partikel so vor eine Nadelspitze platziert, dass eine Nadelspitze bzw. ihre Absaugöffnung verstopft wird. Dieser Zustand wird als Okklusion bezeichnet. In einem solchen Fall steigt der Ansaugdruck innerhalb der Aspirationsleitung deutlich an, wobei eine Emulsifikation in diesem Moment unterbrochen ist. Erst wenn zum Beispiel durch einen sehr starken Energieeintrag, einen deutlichen Anstieg des Ansaugdruckes, oder durch eine Umkehrung der Aspirationspumpenlaufrichtung das Partikel von der Nadelspitze wieder entfernt ist, kann ein übliches Absaugen des Fluides und der kleinen Partikel erfolgen. In einem solchen Moment wird eine Verstopfung somit aufgebrochen, wobei der zuvor anliegende hohe Unterdruck schlagartig abnimmt. Der dadurch entstehende Sog kann dazu führen, dass nicht nur kleine Partikel und Fluid zur Aspirationsnadel gezogen werden, sondern auch ein Teil des Kapselsackes mit der Nadel in Kontakt kommt. Wenn der Kapselsack durchstochen wird, führt dies zu erheblichen Komplikationen für den Patienten, welche unbedingt vermieden werden müssen. Ein erfahrener Operateur hat im Laufe der Zeit ein Gespür dafür entwickelt, wenn eine Okklusion kurz bevorsteht, aufzubrechen. Trotzdem bleibt stets ein Risiko, dass eine Verletzung des Patientenauges auftreten könnte.

In der DE 197 18 139 A1 ist ein Verfahren zur Phakoemulsifikation beschrieben, bei dem Laserwellen mit einem Er:YAG-Laser auf eine mittels dieser Laserstrahlung zu zerkleinernde Augenlinse gerichtet werden.

In WO 2012/041289 A1 ist eine Steuerungsvorrichtung für ein ophthalmochirurgisches System zur Phakoemulsifikation einer Augenlinse beschrieben, welches ein optisches System aufweist, mit dem von einem Objektbereich ein Bild erzeugbar ist, wobei sich in dem Objektbereich mindestens ein Teil der zu emulsifizierenden Augenlinse und ein Teil einer Nadel eines Phakohandstückes mit einer Aspirationsleitung anordnen lässt.

In der WO 2007/073802 A1 ist ein Verfahren zum Beleuchten eines Operationssitus mit Beleuchtungsstrahlung beschrieben, in welchem über wenigstens eine Flüssigkeitsleitung mit einem dem Operationssitus zugewandten distalen Ende eine durch die Flüssigkeitsleitung strömende Flüssigkeit dem Operationssitus zugeführt und/oder vom Operationssitus abgeführt wird.

Es ist eine Aufgabe der Erfindung, eine Steuerungsvorrichtung für ein ophthalmochirurgisches System zur Phakoemulsifikation einer Augenlinse zu schaffen, wobei die Phakoemulsifikation mittels eines Phakohandstückes erfolgt, dem Ultraschallenergie zugeführt wird, wobei bei dem System das Auftreten einer Okklusion an und in einer Phakoemulsifikationsnadel und das Aufbrechen einer derartigen Okklusion an und in einer solchen Nadel schnell und sicher erkannt werden kann, wobei die Phakoemulsifikation mit hoher Effizienz möglich und ein Risiko für eine Verletzung eines Patientenauges gering ist. Ferner ist es eine Aufgabe, ein ophthalmochirurgisches System mit einer derartigen Steuerungsvorrichtung zu schaffen.

Die Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe für das ophthalmochirurgische System wird durch den Gegenstand des unabhängigen Patentanspruchs 4 gelöst.

Die Steuerungsvorrichtung für ein ophthalmochirurgisches System zur Phakoemulsifikation einer Augenlinse, wobei das System eine Aspirationsleitung zum Absaugen von zertrümmerten Linsenpartikel der Augenlinse und von Fluid aufweist, wobei die Aspirationsleitung innerhalb einer Nadel eines Phakohandstückes verläuft und dem Phakohandstück eine Ultraschallenergie für die Zerkleinerung der Augenlinse durch eine Longitudinalschwingung der Nadel zugeführt werden kann, weist auf:
- einen Sender, welcher geeignet ist, elektromagnetische Strahlung abzustrahlen,
- eine Kopplungsvorrichtung, mit welcher sich mindestens ein Teil der abgestrahlten elektromagnetischen Strahlung so zu einem distalen Ende der Kopplungsvorrichtung weiterleiten lässt, dass der mindestens eine Teil der elektromagnetischen Strahlung in die Aspirationsleitung des ophthalmochirurgischen Systems einkoppelbar ist, wobei das distale Ende der Kopplungsvorrichtung außerhalb der Aspirationsleitung angeordnet ist,
- einen Empfänger, welcher geeignet ist, mindestens einen Teil der elektromagnetischen Strahlung zu empfangen,
- und eine Steuerungseinheit, mit welcher sich in Abhängigkeit vom Betrag der empfangenen Strahlung ein Betrag eines Parameters des ophthalmochirurgischen Systems steuern lässt, wobei die elektromagnetische Strahlung eine Wellenlänge von größer als 750 Nanometer aufweist und eine gepulste Infrarotstrahlung mit einer Bestrahlungsstärke von maximal 0,1 W/cm² ist.

Bei der erfindungsgemäßen Steuerungsvorrichtung wird der Umstand genutzt, dass elektromagnetische Strahlung eine sehr schnelle Wellenausbreitungsgeschwindigkeit besitzt. In einem wässrigen Medium, wie dies bei einem Fluid in einer Aspirationsleitung oder Irrigationsleitung vorliegt, beträgt sie etwa 225000 km/s. Wenn die Strahlung in eine Aspirationsleitung eingekoppelt wird, lässt sich somit sehr schnell eine Information in Erfahrung bringen, ob in der Aspirationsleitung eine Okklusion vorliegt und ob diese Okklusion gerade aufbricht. In Abhängigkeit von dem Betrag der empfangenen Strahlung kann dann ein zugehöriges ophthalmochirurgisches System entsprechend gesteuert werden, um eine Gefährdung des Patientenauges gering zu halten.

Bisher übliche ophthalmochirurgische Systeme nutzen zur Erfassung einer Okklusion oder eines Okklusionsdurchbruches den Druckverlauf in einer Aspirationsleitung. Eine Druckwelle pflanzt sich jedoch nur mit Schallgeschwindigkeit, also mit etwa 1,5 km/s fort. Da bei einer Aspirationsleitung häufig elastische Schläuche zum Einsatz kommen, sind für die Weiterleitung des Drucksignals entlang einer Länge von etwa zwei Metern bis zum Drucksensor somit etwa 3 Millisekunden erforderlich. Zur Ermittlung eines Druckgradienten sind gemäß dem Nyquist-Shannon-Abtasttheorem mindestens weitere 3 Millisekunden nötig. Durch die erfindungsgemäße Steuerungsvorrichtung kann das Erkennen eines Okklusionsdurchbruches oder einer Okklusion auf weniger als 0,1 ms reduziert werden. Damit kann wesentlich schneller eine Steuerung eines Betrages eines Parameters des ophthalmochirurgischen Systems eingeleitet werden.

Durch die sehr schnelle Wellenausbreitung der elektromagnetischen Strahlung ist es möglich, den Sender weit von einer Nadel eines Phakoemulsifikations-Handstückes entfernt anzuordnen. Da gemäß der Erfindung die elektromagnetische Strahlung mittels einer Kopplungsvorrichtung in eine Aspirationsleitung einkoppelbar ist, wobei das distale Ende der Kopplungsvorrichtung außerhalb der Aspirationsleitung angeordnet ist, kann das Fluid innerhalb der Aspirationsleitung ungehindert strömen.

Die Einkopplung der elektromagnetischen Strahlung in die Aspirationsleitung kann bevorzugt direkt im Handstück erfolgen. Damit ist es möglich, die Strahlung auf den Bereich der Nadel und die unmittelbar angrenzende Aspirationsleitung zu begrenzen. In diesem Bereich ist der Leitungsquerschnitt am kleinsten und somit die Wahrscheinlichkeit für eine Okklusion am höchsten.

Gemäß der Erfindung weist die elektromagnetische Strahlung eine Wellenlänge von größer als 750 Nanometer auf. Eine solche Strahlung ist vorteilhaft, da sie eine relativ geringe Energie besitzt und somit die Gefahr gering ist, dass dadurch eine Schädigung des Auges auftritt. Gemäß der Erfindung ist die elektromagnetische Strahlung eine gepulste Infrarotstrahlung mit einer Bestrahlungsstärke von maximal 0,1 Watt pro cm². Damit ist sichergestellt, dass auch bei einer Beleuchtung der Netzhaut während einer ophthalmochirurgischen Operation die Netzhaut nicht geschädigt wird. Die elektromagnetische Strahlung dient also nicht dazu, ein Erwärmen oder Erhitzen eines Zielobjektes zu erreichen, sondern ganz im Gegenteil lediglich als Messstrahlung zu wirken, welche gerade so stark ist, dass sie von einem Empfänger noch empfangen werden kann. Mit der elektromagnetischen Strahlung wird keine Linse oder ein Linsenpartikel zertrümmert oder ein Gewebe koaguliert. Eine gepulste Strahlung ist vorteilhaft, da auf diese Weise eventuell in der Umgebung nicht gepulste Strahlung mit ähnlicher Wellenlänge bei der Auswertung des Signals nicht berücksichtigt wird.

Gemäß einer bevorzugten Ausführungsform kann der Empfänger die aus der Aspirationsleitung austretende elektromagnetische Strahlung empfangen. Wenn somit am distalen Ende der Aspirationsleitung kein Linsenpartikel die Aspirationsleitung verstopft, tritt eine vom Sender abgestrahlte elektromagnetische Strahlung aus der Aspirationsleitung heraus, so dass der Empfänger noch relativ viel elektromagnetische Strahlung empfangen kann. Bei einer Okklusion innerhalb der Aspirationsleitung oder an der Nadelspitze tritt hingegen keine elektromagnetische Strahlung mehr aus dem distalen Ende der Aspirationsleitung aus, so dass ein Empfänger auch keine Strahlung erfassen kann. Wird dann durch erhöhten Ansaugdruck oder zunehmende Longitudinalschwingung der Nadel des Phakohandstückes das Linsenpartikel in kleinere Bruchstücke zertrümmert, kann am distalen Ende der Aspirationsleitung wieder elektromagnetische Strahlung austreten, welche vom Empfänger detektiert werden kann. Dieser Unterschied in der Signalstärke der empfangbaren elektromagnetischen Strahlung ist ein Indikator dafür, dass ein Okklusionsdurchbruch stattgefunden hat.

Gemäß einer weiteren Ausführungsform der Erfindung kann der Empfänger die von einem Linsenpartikel reflektierte elektromagnetische Strahlung empfangen. Falls ein Linsenpartikel ein distales Ende einer Aspirationsleitung, also die Nadelspitze, vollständig verstopft, kann nahezu keine elektromagnetische Strahlung mehr aus dem distalen Ende der Aspirationsleitung austreten. Sämtliche von einem Sender abgestrahlte und in die Aspirationsleitung eingekoppelte elektromagnetische Strahlung wird dann von dem die Aspirationsleitung verstopfenden Linsenpartikel reflektiert. Der Empfänger der erfindungsgemäßen Steuerungsvorrichtung muss dann so angeordnet sein, dass er diese vom Linsenpartikel reflektierte Strahlung empfängt. Falls durch erhöhten Ansaugdruck oder durch Schwingung der Nadel des Phakohandstückes das Linsenpartikel zerbricht, kann nur noch ein Teil der ausgesendeten elektromagnetischen Strahlung von diesem Bruchstück reflektiert werden, so dass im Falle eines Okklusionsdurchbruches der Empfänger eine geringere elektromagnetische Strahlung empfangen kann. Der Betrag der reflektierten elektromagnetischen Strahlung ist somit ein Indikator dafür, ob eine Okklusion oder ein Okklusionsdurchbruch stattgefunden hat.

Die Aspirationsleitung weist vorzugsweise eine Leitungsinnenwand auf, welche die in die Aspirationsleitung eingekoppelte elektromagnetische Strahlung reflektiert. Fluid in der Aspirationsleitung kann als Leiter für die elektromagnetische Strahlung dienen, wobei eine Reflektion der Strahlung an der Leitungsinnenwand dazu führt, dass ein relativ großer Betrag der zugeführten elektromagnetischen Strahlung bis zum distalen Ende der Aspirationsleitung geleitet werden kann.

Die Aspirationsleitung kann auch eine Leitungswand aufweisen, welche darin eingekoppelte elektromagnetische Strahlung weiterleitet. Die Weiterleitung der elektromagnetischen Strahlung kann entweder durch den Werkstoff der Aspirationsleitung selbst oder durch einen Lichtleiter in der Wand der Aspirationsleitung erfolgen. Eine solche Ausführungsform ist vorteilhaft, da somit kein Fluid oder in der Aspirationsleitung vorhandene Linsenpartikel die Weiterleitung der elektromagnetischen Strahlung behindern können.

Die erfindungsgemäße Steuerungsvorrichtung weist eine Steuerungseinheit auf, mit welcher sich in Abhängigkeit vom Betrag der empfangenen Strahlung ein Betrag eines Parameters des ophthalmochirurgischen Systems steuern lässt. Der Parameter des ophthalmochirurgischen System kann eine einem Phakohandstück durch eine Energieversorgung zugeführte Ultraschallenergie, eine Saugleistung einer Aspirationspumpe, oder ein Volumenstrom eines Fluides zur Belüftung der Aspirationsleitung sein. Die Steuerungseinheit kann somit bewirken, dass im Falle eines Okklusionsdurchbruches die dem Phakohandstück zugeführte Ultraschallenergie abgeschaltet wird, so dass die Gefahr einer Beschädigung des Kapselsackes verringert wird. Alternativ oder zusätzlich kann die Steuerungseinheit im Falle eines Okklusionsdurchbruches eine Saugleistung einer Aspirationspumpe reduzieren oder vollkommen abschalten, so dass der Unterdruck in der Aspirationsleitung nicht mehr auf dem vorhandenen Niveau aufrechterhalten wird. Alternativ oder zusätzlich kann die Steuerungseinheit ein Fluid in die Aspirationsleitung zuführen, somit belüften, um damit den Unterdruck aktiv zu verringern.

Vorzugsweise kann der Betrag des Parameters des ophthalmochirurgischen Systems in Abhängigkeit vom Druck in der Aspirationsleitung gesteuert werden. Es kann zum Beispiel vorgesehen sein, dass die Steuerungsvorrichtung erst zum Einsatz kommt, wenn der Unterdruck in der Aspirationsleitung ein vorbestimmtes Niveau erreicht hat. Damit lassen sich Artefakte und ungefährliche Druckschwankungen ausfiltern, so dass es zu keinen hektischen und unnötigen Eingriffen der Steuerungsvorrichtung in das ophthalmochirurgische System kommt.

Das ophthalmochirurgische System zur Phakoemulsifikation einer Augenlinse weist eine Steuerungsvorrichtung wie vorstehend beschrieben und eine Systemkomponenteneinheit auf, welche eine Energieversorgung für ein Phakohandstück und/oder eine Aspirationspumpe und/oder einen Refluxbehälter zur Aufnahme von Fluid zur Belüftung der Aspirationsleitung aufweist.

Die Erfindung wird durch die Ansprüche definiert. Weitere Vorteile und Merkmale der Erfindung werden mit Bezug auf die nachfolgenden Zeichnungen erklärt, in welchen zeigen:
- Figur 1: eine schematische Darstellung eines ophthalmochirurgischen Systems mit einer Steuerungsvorrichtung gemäß einer ersten Ausführungsform der Erfindung;
- Figur 2: eine schematische Darstellung einer Aspirationsleitung und eines Empfängers, wenn keine Okklusion der Aspirationsleitung vorliegt;
- Figur 3: eine schematische Darstellung von Signalverläufen eines Senders und eines Empfängers bei einer Anordnung gemäß Figur 2;
- Figur 4: eine schematische Darstellung einer Aspirationsleitung und eines Empfängers im Falle einer Okklusion;
- Figur 5: eine schematische Darstellung von Signalverläufen eines Senders und eines Empfängers bei der in Figur 4 dargestellten Situation;
- Figur 6: eine schematische Darstellung der Signalverläufe eines Senders und Empfängers in Abhängigkeit von unterschiedlichen Okklusionszuständen;
- Figur 7: eine schematische Darstellung eines ophthalmochirurgischen Systems mit einer Steuerungsvorrichtung gemäß einer zweiten Ausführungsform der Erfindung;
- Figur 8: eine schematische Darstellung eines ophthalmochirurgischen Systems mit einer Steuerungsvorrichtung gemäß einer dritten Ausführungsform der Erfindung, wenn keine Okklusion vorliegt;
- Figur 9: eine schematische Darstellung von Signalverläufen von Sender und Empfänger für eine Ausführungsform gemäß Figur 8;
- Figur 10: eine schematische Darstellung eines Systems gemäß Fig. 8, wenn Okklusion vorliegt;
- Figur 11: eine schematische Darstellung von Signalverläufen von Sender und Empfänger für die Situation gemäß Figur 10;
- Figur 12: eine schematische Darstellung von Signalverläufen von Sender und Empfänger in Abhängigkeit von unterschiedlichen Okklusionszuständen unter Berücksichtigung des Druckverlaufes in einer Aspirationsleitung;
- Figur 13: eine Querschnittsansicht einer Aspirationsleitung, welche elektromagnetische Strahlung mittels Fluid weiterleitet;
- Figur 14: eine Querschnittsansicht einer Aspirationsleitung, welche eine Leitungsinnenwand aufweist, welche die in die Aspirationsleitung eingekoppelte elektromagnetische Strahlung reflektiert;
- Figur 15: eine Querschnittsansicht einer Aspirationsleitung, welche eine Leitungswand aufweist, welche darin eingekoppelte elektromagnetische Strahlung weiterleitet; und
- Figur 16: eine schematische Darstellung eines ophthalmochirurgischen Systems mit einer Steuerungsvorrichtung gemäß einer vierten Ausführungsform der Erfindung.

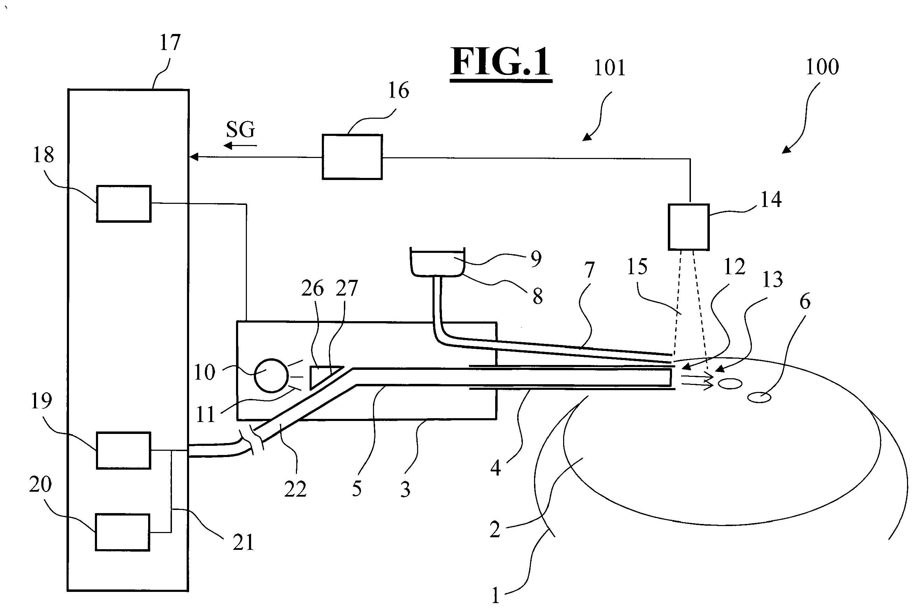

In Figur 1 ist ein ophthalmochirurgisches System 100 mit einer Steuerungsvorrichtung 101 dargestellt. Ein Auge 1 besitzt eine zu emulsifizierende Augenlinse 2, wobei ein Phakohandstück 3 mit einer Nadel 4 in die Linse 2 injiziert wurde. Innerhalb der Nadel 4 verläuft eine Aspirationsleitung 5, mit der zertrümmerte Linsenpartikel 6 und Fluid abgesaugt werden können. Zusätzlich wird mittels einer Irrigationsleitung 7 aus einem Irrigationsfluidbehälter 8 ein Irrigationsfluid 9 zur Spitze der Nadel 4 geleitet. Ein Sender 10, welcher z. B. innerhalb des Phakohandstückes 3 angeordnet sein kann, sendet elektromagnetische Strahlung 11 zu einer Kopplungsvorrichtung 26 aus. Die Kopplungsvorrichtung 26 ist geeignet, mindestens einen Teil der abgestrahlten elektromagnetischen Strahlung 11 aufzunehmen und so zu einem distalen Ende 27 der außerhalb der Aspirationsleitung 5 angeordneten Kopplungsvorrichtung 26 weiterzuleiten, so dass der mindestens eine Teil der elektromagnetischen Strahlung 11 in die Aspirationsleitung 5 einkoppelbar ist. Bei der in Fig. 1 dargestellten Ausführungsform wird die elektromagnetische Strahlung 11 von dem distalen Ende 27 ausgehend in eine gerade Strecke der Aspirationsleitung 5 eingekoppelt. Die Strahlung 11 kann jedoch auch entlang einer gekrümmten oder mit Knicken versehenen Aspirationsleitung 5 eingekoppelt und von dort weitergeleitet werden, wobei der Sender 10 außerhalb des Handstückes angeordnet ist. Am distalen Ende 12 der Aspirationsleitung 5 tritt dann die elektromagnetische Strahlung aus, siehe Bezugszeichen 13, wenn das distale Ende 12 der Aspirationsleitung 5 von Partikeln 6 nicht verstopft ist.

Bei dieser ersten Ausführungsform ist ein Empfänger 14 so angeordnet, dass er die am distalen Ende 12 austretende elektromagnetische Strahlung erfassen kann. Der Empfänger 14 besitzt dazu einen Empfangsbereich 15, welcher mindestens eine Zone um das distale Ende 12 der Aspirationsleitung 5 beinhaltet. Die vom Empfänger 14 empfangene Strahlung wird derart verarbeitet, dass bestimmt wird, ob eine Okklusion vorhanden ist oder nicht und ob eine Okklusion beginnt oder gerade durchbricht. Diese Information wird an eine Steuerungseinheit 16 geleitet, welche in Abhängigkeit von dieser Information eine Steuergröße SG an eine Systemkomponenteneinheit 17 abgibt.

Die Systemkomponenteneinheit 17 kann eine Energieversorgung 18 aufweisen, welche wiederum mit dem Phakohandstück 3 verbunden ist. Wenn mittels des Empfängers 14 festgestellt wird, dass ein Okklusionsdurchbruch stattgefunden hat, kann die Steuerungseinheit 16 die Energieversorgung 18 so ansteuern, dass die Nadel 4 zu keinen Ultraschallschwingungen mehr angeregt wird. Damit erfolgt keine weitere Energiezufuhr, so dass die Gefahr einer Beschädigung der Linsenkapsel geringer ist. Die Systemkomponenteneinheit 17 kann auch eine Aspirationspumpe 19 aufweisen, welche von der Steuerungseinheit 16 angesteuert wird, wobei die Aspirationspumpe 19 mit der Aspirationsleitung 5, welche ein Aspirationsfluid 22 enthält, verbunden ist. Im Falle eines Okklusionsdurchbruches kann somit die Aspirationspumpe 19 gestoppt oder ihre Drehrichtung umgekehrt werden. Ferner kann die Systemkomponenteneinheit 17 auch einen Refluxbehälter 20 aufweisen, welcher von der Steuerungseinheit 16 angesteuert wird, so dass mittels einer Refluxleitung 21 ein Volumenstrom eines Fluides zur Belüftung der Aspirationsleitung 5 zugeführt wird.

Die Kopplungsvorrichtung 26 kann ferner einen optischen Lichtleiter, beispielsweise eine Lichtleitfaser, ausgeführt als Monomode- oder Multimode-Faser, oder eine Linse aufweisen.

Ist die Aspirationsleitung für elektromagnetische Strahlung durchlässig, kann die Einkopplung der elektromagnetischen Strahlung an jeder beliebigen Stelle der Aspirationsleitung erfolgen. Falls die Aspirationsleitung nicht für elektromagnetische Strahlung durchlässig sein sollte, kann die Aspirationsleitung mit einer für elektromagnetische Strahlung durchlässigen und lokal begrenzten Zone versehen sein.

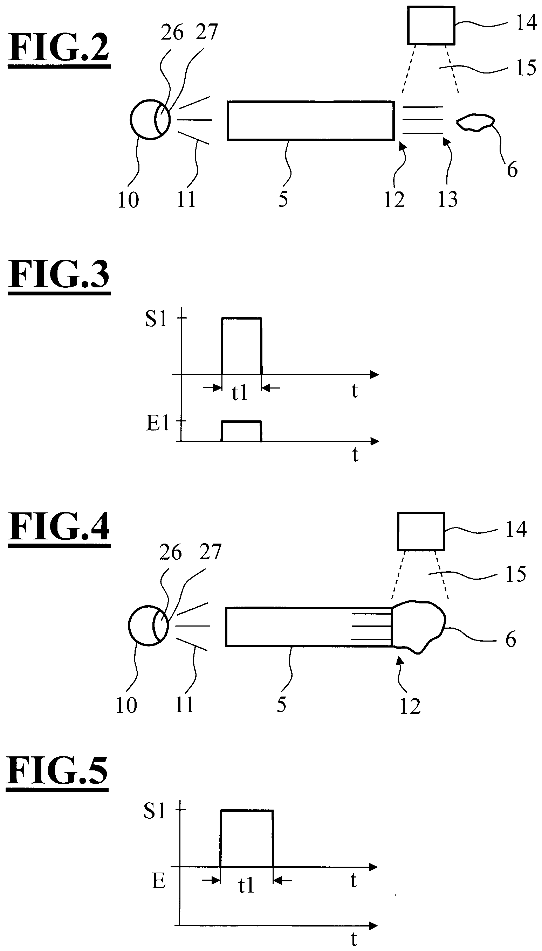

Figur 2 zeigt in einer schematischen stark vereinfachten Darstellung die Situation, wenn aus der Aspirationsleitung 5 eine elektromagnetische Strahlung am distalen Ende 12 austritt, wobei ein Partikel 6 das distale Ende 12 der Aspirationsleitung 5 nicht verstopft. Die elektromagnetische Strahlung 11 wird von einem Sender 10 mittels einer optischen Linse als Kopplungsvorrichtung 26 abgestrahlt. Bei der in Fig. 2 gezeigten Ausführungsform kann der Empfänger 14 die innerhalb seines Empfangsbereiches 15 austretende elektromagnetische Strahlung, siehe Bezugszeichen 13, empfangen.

In Figur 3 sind Signalverläufe dargestellt, welche bei der in Fig. 2 gezeigten Situation zu erwarten sind. Der Sender 10 sendet während einer Zeit t1 eine elektromagnetische Strahlung 11 mit der Strahlstärke S1 aus. Der Empfänger 14 empfängt nahezu ohne Zeitverzögerung eine am distalen Ende 12 austretende elektromagnetische Strahlung mit einer Strahlstärke E1, welche niedriger als die abgestrahlte Signalstärke S1 ist. Die Signalstärke E1 ist niedriger als die Signalstärke S1, da die vom Sender 10 abgestrahlte elektromagnetische Strahlung 11 auf dem Weg durch die Aspirationsleitung 5 an Intensität verliert.

Figur 4 zeigt eine Situation analog zu Figur 2, jedoch in dem Zustand, wenn die Aspirationsleitung 5 am distalen Ende 12 mit einem Linsenpartikel 6 verstopft ist. In diesem Fall tritt aus der Aspirationsleitung 5 keine elektromagnetische Strahlung aus, so dass der Empfänger 14 innerhalb seines Empfangsbereiches 15 keine elektromagnetische Strahlung empfangen kann.

Figur 5 zeigt Signalverläufe für den Sender 10 und den Empfänger 14 für die in Fig. 4 gezeigte Situation. Während der Sender 10 eine elektromagnetische Strahlung 11 mit einer Strahlstärke S1 abgibt, kann der Empfänger keine elektromagnetische Strahlung empfangen.

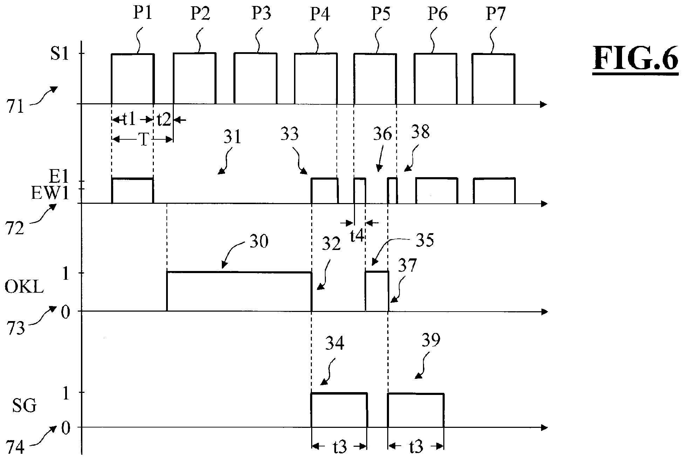

Figur 6 zeigt mehrere Diagramme in Abhängigkeit von der Zeit. Diagramm 71 zeigt Signalverläufe für einen Sender 10, Diagramm 72 zeigt Signalverläufe für einen Empfänger 14, Diagramm 73 zeigt Signalverläufe von vorhandenen oder nicht vorhandenen Okklusionszuständen der Aspirationsleitung, und Diagramm 74 zeigt Verläufe einer Steuergröße SG. Es wird angenommen, dass während einer Zeitdauer t1 der Sender eine elektromagnetische Strahlung mit einer Strahlstärke S1 aussendet. Während der sich daran anschließenden Zeitdauer t2 wird vom Sender keine elektromagnetische Strahlung abgegeben, wobei sich dieses Muster mit den Zeitdauern t1 und t2 fortwährend wiederholt. Vom Sender wird somit eine gepulste Strahlung mit einer Periodendauer T abgegeben. Die Strahlung erfolgt also während Pulsphasen, in Fig. 6 mit P1, P2 , P3, P4, P5, P6, P7 bezeichnet, welche jeweils durch Pulspausen unterbrochen werden. Während der ersten Pulsphase P1 mit der Zeitdauer t1 empfängt der Empfänger eine elektromagnetische Strahlung mit der Signalstärke E1. Während der anschließenden Zeitdauer t2, in welcher der Sender keine elektromagnetische Strahlung abgibt, wird angenommen, dass das distale Ende der Aspirationsleitung von einem Linsenpartikel so verstopft wird, dass eine Okklusion eintritt, siehe Bezugszeichen 30 in Diagramm 73. Während der zweiten Pulsphase P2 des Senders kann der Empfänger somit keine elektromagnetische Strahlung empfangen, siehe Bezugszeichen 31 in Diagramm 72. Es wird angenommen, dass diese Situation noch während der dritten Pulsphase P3 vorliegt. Während der vierten Pulsphase P4 kommt es jedoch zu einem Okklusionsdurchbruch, siehe Bezugszeichen 32 in Diagramm 73, so dass unmittelbar danach der Empfänger wieder eine elektromagnetische Strahlung mit einer Signalstärke oberhalb einer vorbestimmten Schwelle EW1 empfangen kann, siehe Bezugszeichen 33 in Diagramm 72.

Dieser Anstieg der Signalstärke auf die Höhe E1 ist ein sicherer Indikator dafür, dass ein Okklusionsdurchbruch stattgefunden hat. Die Steuerungseinheit gibt bei der erfindungsgemäßen Steuerungsvorrichtung eine Steuergröße SG an eine Systemkomponenteneinheit ab, siehe Bezugszeichen 34 in Diagramm 74, so dass sich ein Betrag eines Parameters des ophthalmochirurgischen Systems steuern lässt. Es wird angenommen, dass die Steuergröße SG während einer vorbestimmten Zeitdauer t3 anliegt.

Zu Beginn der anschließenden Pulsphase P5 ist noch keine erneute Okklusion vorhanden. Während der Pulsphase P5 wird angenommen, dass eine solche Okklusion jedoch wieder eintritt, siehe Bezugszeichen 35 in Diagramm 73. Dies wird daran erkannt, dass der Empfänger nur kurzzeitig während einer Zeitdauer t4 ein Signal mit der Empfangsstärke E1 empfangen kann. Während der Dauer der Okklusion 35 ist kein Signal vom Empfänger erfassbar, siehe Bezugszeichen 36 in Diagramm 72. Wenn dann während der Pulsphase P5 die Okklusion durchbricht, siehe Bezugszeichen 37 in Diagramm 73, wird vom Empfänger wieder ein Signal mit der Signalstärke E1 erfasst, siehe Bezugszeichen 38 in Diagramm 72. Dieser Anstieg der Signalstärke des Empfängers ist ein sicherer Indikator dafür, dass ein Okklusionsdurchbruch stattgefunden hat, so dass die Steuerungseinheit erneut eine Steuergröße SG, siehe Bezugszeichen 39 in Diagramm 74, an die Systemkomponenteneinheit abgibt, woraufhin sich ein Betrag eines Parameters des ophthalmochirurgischen Systems steuern lässt. Es wird angenommen dass die Steuergröße SG während einer Zeitdauer t3 anliegt. Bei den darauf folgenden Pulsphasen P6 und P7 sei angenommen, dass keine Okklusion auftritt, so dass der Empfänger während der gesamten anliegenden Sendedauer jeweils eine Signalstärke in der Höhe E1 empfangen kann.

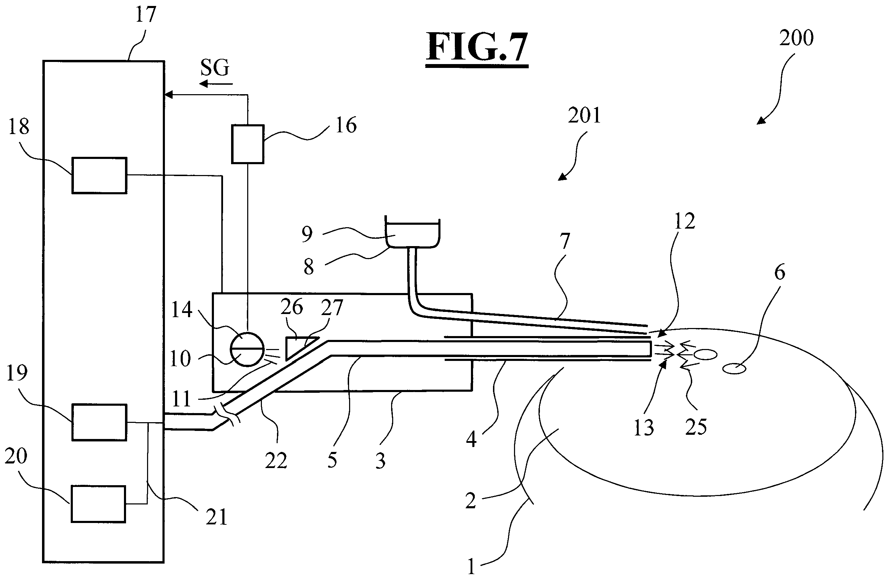

In Figur 7 ist ein ophthalmochirurgisches System 200 mit einer Steuerungsvorrichtung 201 gemäß einer zweiten Ausführungsform der Erfindung dargestellt. Diese zweite Ausführungsform unterscheidet sich von der in Figur 1 dargestellten ersten Ausführungsform dadurch, dass der Empfänger 14 nicht mehr die am distalen Ende 12 austretende elektromagnetische Strahlung, siehe Bezugszeichen 13, erfasst, sondern eine von Linsenpartikeln 6 reflektierte Strahlung 25 erfasst.

Vom Sender 10 wird mittels einer Kopplungsvorrichtung 26 eine elektromagnetische Strahlung 11 in eine Aspirationsleitung 5 eingekoppelt. Wenn keine Okklusion vorliegt, siehe Figur 7, kann ein Teil der am distalen Ende der Aspirationsleitung 5 austretenden elektromagnetischen Strahlung, siehe Bezugszeichen 13, von einem Linsenpartikel 6 als Reflektionsstrahlung 25 reflektiert werden.

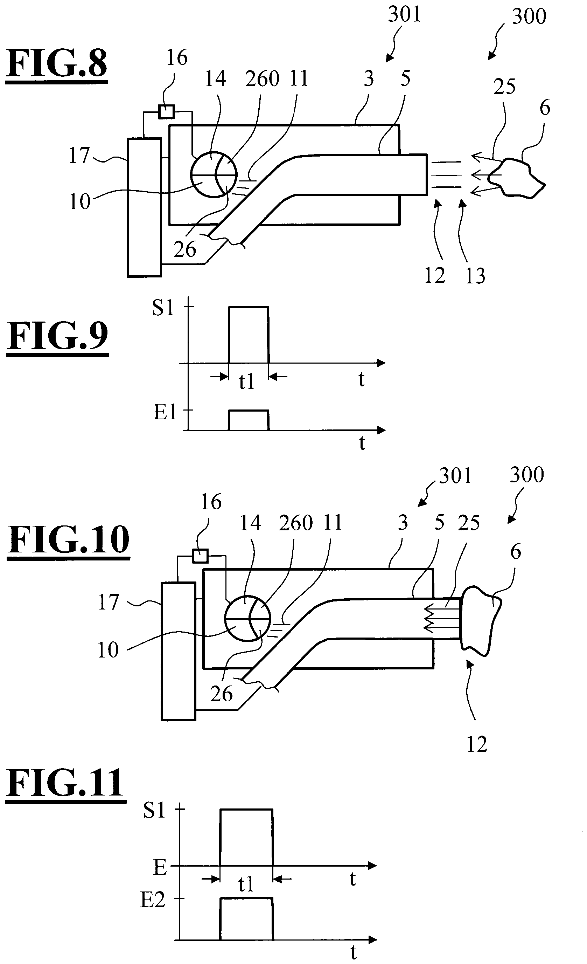

Bei der in Fig. 7 dargestellten Ausführungsform wird ein einziges Kopplungselement 26 verwendet, um elektromagnetische Strahlung 11 in die Aspirationsleitung einzukoppeln und reflektierte Strahlung 25 aus der Aspirationsleitung 5 zum Empfänger 14 auszukoppeln. Bei der in Fig. 8 dargestellten dritten Ausführungsform einer Steuerungsvorrichtung 301 eines ophthalmochirurgischen Systems 300 empfängt ein Empfänger 14, welcher nahe am Sender 10 angeordnet sein kann, mindestens einen Teil der von den Linsenpartikeln 6 reflektierten Strahlung 25 mittels einer von der Kopplungsvorrichtung 26 getrennten zweiten Kopplungsvorrichtung 260.

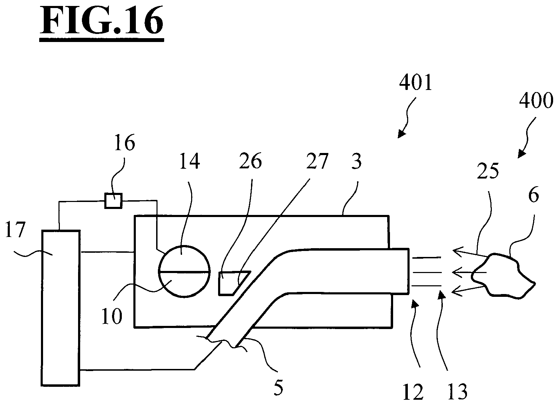

Figur 16 zeigt eine schematische Darstellung einer vierten Ausführungsform einer erfindungsgemäßen Steuerungsvorrichtung 401 eines ophthalmochirurgischen Systems 400. Hierbei kann die Kopplungsvorrichtung 26 so gestaltet sein, dass lediglich die vom Sender 10 abgestrahlte elektromagnetische Strahlung mittels der Kopplungsvorrichtung 26 weitergeleitet und über das distale Ende 27 in die Aspirationsleitung 5 eingekoppelt wird. Die von den Linsenpartikeln 6 reflektierte Strahlung 25 dringt in die Aspirationsleitung 5 ein und wird direkt von dem Empfänger 14 ohne eine davor angeordnete Kopplungsvorrichtung erfasst. Dies ist vorteilhaft, wenn am Empfänger nur noch eine reflektierte Strahlung 25 mit sehr geringer Strahlstärke bzw. Intensität eintrifft, da somit kein weiteres Bauelement die eintreffende Strahlung 25 in ihrer Strahlstärke weiter schwächen kann.

In Figur 9 sind schematisch zugehörige Signalverläufe für diese Situation gemäß Figur 8 dargestellt. Ein Sender 10 sendet während einer Zeit t1 eine elektromagnetische Strahlung mit einer Signalstärke S1 aus, wobei ein Empfänger 14 eine elektromagnetische Strahlung mit einer Signalstärke E1, welche kleiner als S1 ist, empfängt.

Figur 10 zeigt die Situation bei einer Okklusion der Aspirationsleitung bei der dritten Ausführungsform der Erfindung. In diesem Fall wird sämtliche in der Aspirationsleitung 5 vorhandene elektromagnetische Strahlung 11 vom Linsenpartikel 6 als Reflektionsstrahlung 25 reflektiert, so dass keine elektromagnetische Strahlung am distalen Ende 12 der Aspirationsleitung austritt.

Figur 11 zeigt die zu der in Fig. 10 dargestellten Situation zugehörigen Signalverläufe. Wenn der Sender während einer Zeit t1 eine elektromagnetische Strahlung mit der Signalstärke S1 aussendet, kann bei vollständiger Reflektion am Linsenpartikel 6 eine elektromagnetische Strahlung mit einer Signalstärke E2 empfangen werden. Man kann davon ausgehen, dass bei vollständiger Reflektion die Signalstärke E2 größer als E1 ist.

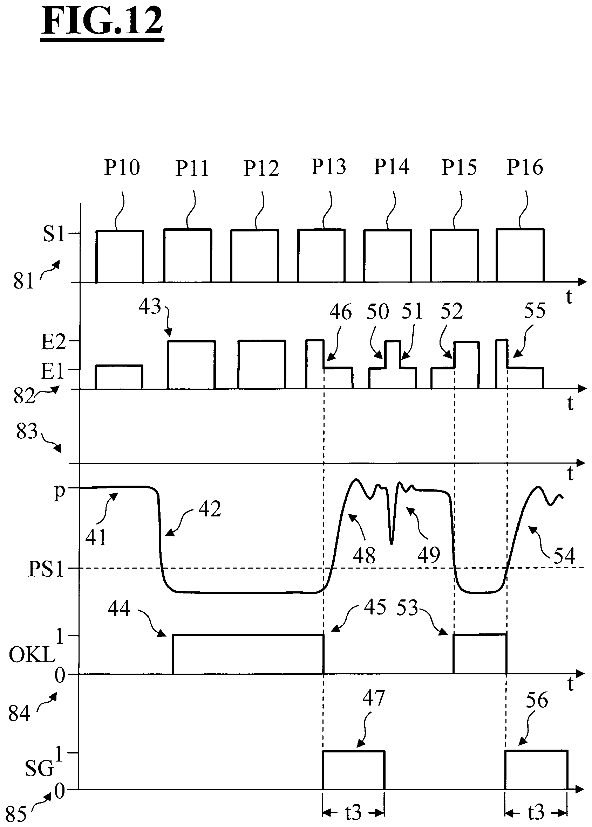

Figur 12 zeigt für eine Steuerungsvorrichtung gemäß der zweiten Ausführungsform mehrere Diagramme in Abhängigkeit von der Zeit. Diagramm 81 zeigt Signalverläufe für einen Sender 10, Diagramm 82 zeigt Signalverläufe für einen Empfänger 14, Diagramm 83 zeigt einen Signalverlauf eines Druckes in der Aspirationsleitung, Diagramm 84 zeigt Signalverläufe bei vorhandenen oder nicht vorhandenen Okklusionszuständen der Aspirationsleitung, und Diagramm 85 zeigt Verläufe einer Steuergröße SG. Während einer ersten Pulsphase P10 sendet ein Sender eine elektromagnetische Strahlung mit einer Signalstärke S1 aus. Wenn keine Okklusion vorliegt, empfängt bei einer Steuerungsvorrichtung gemäß der zweiten Ausführungsform der Erfindung der Empfänger eine elektromagnetische Strahlung mit einer relativ niedrigen Signalstärke E1. In der Aspirationsleitung besteht während dieser Zeit nur ein relativ geringer Unterdruck, siehe Bezugszeichen 41 in Diagramm 83. Wird angenommen, dass daraufhin die Aspirationsleitung verstopft, steigt der Unterdruck in der Aspirationsleitung an, siehe Bezugszeichen 42 in Diagramm 83. Wird eine vorbestimmte Druckschwelle PS1 überschritten, wird überprüft, ob in der darauf folgenden Pulsphase P11 eine Änderung der Signalstärke von E1 auf eine höhere Signalstärke E2 vorliegt. Gemäß Bezugszeichen 43 in Diagramm 82 ist dies der Fall, so dass erkannt wird, dass der Beginn einer Okklusion der Aspirationsleitung vorliegt, siehe Bezugszeichen 44 in Diagramm 84. Es wird angenommen, dass auch bei der darauffolgenden Pulsphase P12 und während eines Teils der darauffolgenden Pulsphase P13 die Okklusion noch vorhanden ist. Wird ferner angenommen, dass während der Pulsphase P13 die Okklusion durchbricht, siehe Bezugszeichen 45 in Diagramm 84, kann dies von der erfindungsgemäßen Steuerungsvorrichtung dadurch erkannt werden, dass die Signalstärke am Empfänger vom Betrag E2 auf E1 absinkt, siehe Bezugszeichen 46 in Diagramm 82. Diese Änderung in der Signalstärke bewirkt, dass die Steuerungseinheit 16 eine Steuergröße SG an eine Systemkomponenteneinheit 17 abgibt, siehe Bezugszeichen 47 in Diagramm 85, wobei die Steuergröße zum Beispiel während einer Zeit t3 anliegt. Der Druckverlauf in der Aspirationsleitung sinkt nach dem Okklusionsdurchbruch deutlich ab, siehe Bezugszeichen 48 in Diagramm 83. Wird ferner angenommen, dass daraufhin zum Beispiel während der anschließenden Pulsphase P14 kurzzeitige Druckschwankungen auftreten, siehe Bezugszeichen 49 in Diagramm 83, welche jedoch unterhalb der vorbestimmten Druckschwelle PS1 liegen, kann trotz einer Änderung der Signalstärke von E1 auf E2, siehe Bezugszeichen 50 in Diagramm 82, oder von E2 auf E1, siehe Bezugszeichen 51 in Diagramm 82, diese Schwankung noch als Artefakt interpretiert werden, so dass von der Steuerungseinheit 16 noch keine Steuergröße SG abgegeben wird. Wenn jedoch der Druckverlauf die Druckschwelle PS1 übersteigt, da z. B. eine Okklusion wieder eintritt, steigt während einer Pulsphase P15 die Signalstärke des Empfängers von E1 auf E2 wieder an, siehe Bezugszeichen 52 in Diagramm 82, so dass der Beginn einer Okklusion erfasst wird, siehe Bezugszeichen 53 in Diagramm 84. Wenn während der darauffolgenden Pulsphase P16 die Okklusion durchbricht, so dass der Druck in der Aspirationsleitung wieder abnimmt, siehe Bezugszeichen 54 in Diagramm 83, sinkt die Signalstärke des Empfängers von E2 auf E1 wieder ab, siehe Bezugszeichen 55 in Diagramm 82, woraufhin die Steuerungseinheit 16 eine Steuergröße SG an eine Systemkomponenteneinheit 17 abgibt, siehe Bezugszeichen 56 in Diagramm 85. Diese Steuergröße SG kann wieder während einer Zeitdauer t3 anliegen.

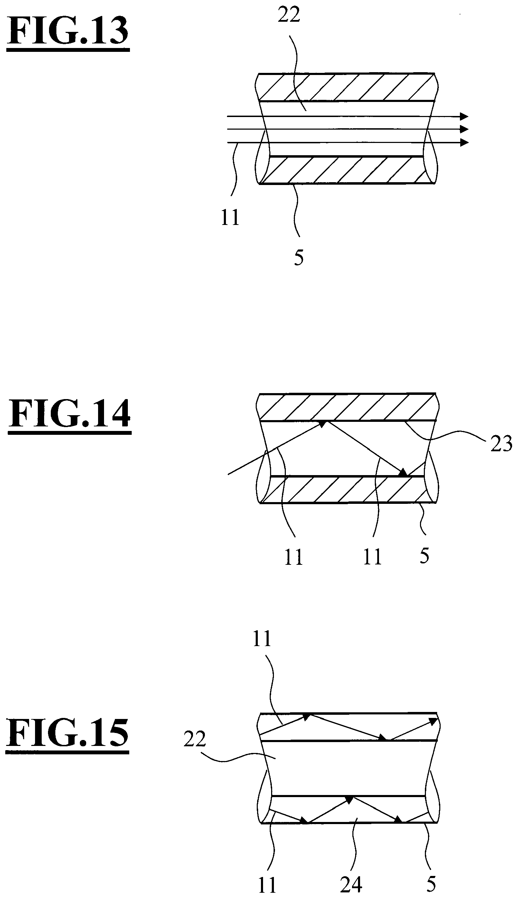

Die Figuren 13, 14 und 15 zeigen unterschiedliche Konstruktionen von Aspirationsleitungen 5. In Figur 13 ist eine Querschnittsansicht einer Aspirationsleitung 5 dargestellt, bei der die elektromagnetische Strahlung 11 durch ein in der Aspirationsleitung 5 vorhandenes Fluid 22 geleitet wird. Zusätzlich oder alternativ dazu kann die elektromagnetische Strahlung 11 auch an einer Innenwand 23 der Aspirationsleitung 5 reflektiert werden, so dass das Weiterleiten der elektromagnetischen Strahlung durch Reflektion erfolgt, siehe Figur 14. Eine weitere Konstruktion der Aspirationsleitung ist in Figur 15 gezeigt, bei welcher eine Leitungswand 24 eine Reflektion einer eingekoppelten elektromagnetischen Strahlung 11 innerhalb der Leitungswand 24 ermöglicht.

## Patentansprüche

1. Steuerungsvorrichtung (101; 201; 301; 401) für ein ophthalmochirurgisches System (100; 200; 300; 400) zur Phakoemulsifikation einer Augenlinse (2), wobei das System (100; 200; 300; 400) eine Aspirationsleitung (5) zum Absaugen von zertrümmerten Linsenpartikeln (6) der Augenlinse (2) und von Fluid aufweist, wobei die Aspirationsleitung (5) innerhalb einer Nadel (4) eines Phakohandstückes (3) verläuft und dem Phakohandstück (3) eine Ultraschallenergie für die Zertrümmerung von Linsenpartikeln in kleinere Bruchstücke durch eine Longitudinalschwingung der Nadel zugeführt werden kann, wobei die Steuerungsvorrichtung aufweist:
- einen Sender (10), welcher geeignet ist, elektromagnetische Strahlung (11) abzustrahlen,
- eine Kopplungsvorrichtung (26), eingerichtet um mindestens einen Teil der abgestrahlten elektromagnetischen Strahlung (11) zu einem distalen Ende (27) der Kopplungsvorrichtung (26) weiterzuleiten und weiterhin eingerichtet, den mindestens einen Teil der elektromagnetischen Strahlung (11) in eine Aspirationsleitung (5) des ophthalmochirurgischen Systems (100; 200; 300; 400) einzukoppeln wobei das distale Ende (27) der Kopplungsvorrichtung eingerichtet ist, (26) außerhalb einer Aspirationsleitung (5) angeordnet zu werden,
- einen Empfänger (14), welcher geeignet ist, mindestens einen Teil der elektromagnetischen Strahlung (13; 25) zu empfangen,
- eine Steuerungseinheit (16), mit welcher sich in Abhängigkeit vom Betrag der empfangenen Strahlung (13; 25) ein Betrag eines Parameters des ophthalmochirurgischen Systems (100; 200; 300; 400) steuern lässt, wobei die elektromagnetische Strahlung (11) eine Wellenlänge von größer als 750 Nanometer aufweist **dadurch gekennzeichnet, dass** die elektromagnetische Strahlung eine gepulste Infrarotstrahlung mit einer Bestrahlungsstärke von maximal 0,1 W/cm² ist.

2. Vorrichtung (101; 201; 301; 401) nach Anspruch 1, wobei der Empfänger (14) eingerichtet ist, die aus einer Aspirationsleitung (5) austretende elektromagnetische Strahlung (13) zu empfangen.

3. Vorrichtung (101; 201; 301; 401) nach Anspruch 1, wobei der Empfänger (14) eingerichtet ist, die von einem Linsenpartikel (6) reflektierte elektromagnetische Strahlung (25) zu empfangen.

4. Ophthalmochirurgisches System (100; 200; 300; 400) zur Phakoemulsifikation mit einer Steuerungsvorrichtung (101; 201; 301; 401) nach einem der vorherigen Ansprüche und einer Systemkomponenteneinheit (17), welche eine Energieversorgung (18) für ein Phakohandstück (3), und/oder eine Aspirationspumpe (19), und/oder einen Refluxbehälter (20) zur Aufnahme von Fluid zur Belüftung der Aspirationsleitung (5) aufweist.

## Claims

1. Control device (101; 201; 301; 401) for an ophthalmic surgical system (100; 200; 300; 400) for phacoemulsification of an eye lens (2), wherein the system (100; 200; 300; 400) comprises an aspiration line (5) for aspirating shattered lens particles (6) of the eye lens (2) and for aspirating fluid, wherein the aspiration line (5) runs inside a needle (4) of a phaco handpiece (3) and ultrasound energy can be supplied to the phaco handpiece (3) for shattering lens particles into smaller fragments by means of a longitudinal vibration of the needle, wherein the control device comprises:
- a transmitter (10), which is suitable for emitting electromagnetic radiation (11),
- a coupling device (26), configured to pass on at least one portion of the emitted electromagnetic radiation (11) to a distal end (27) of the coupling device (26) and furthermore configured to couple the at least one portion of the electromagnetic radiation (11) into an aspiration line (5) of the ophthalmic surgical system (100; 200; 300; 400), wherein the distal end (27) of the coupling device (26) is configured to be arranged outside an aspiration line (5),
- a receiver (14), which is suitable for receiving at least one portion of the electromagnetic radiation (13; 25),
- a control unit (16), with which an absolute value of a parameter of the ophthalmic surgical system (100; 200; 300; 400) can be controlled depending on the absolute value of the received radiation (13; 25), wherein the electromagnetic radiation (11) comprises a wavelength of greater than 750 nanometres, **characterized in that** the electromagnetic radiation is pulsed infrared radiation having an irradiance of a maximum of 0.1 W/cm².

2. Device (101; 201; 301; 401) according to Claim 1, wherein the receiver (14) is configured for receiving the electromagnetic radiation (13) exiting from an aspiration line (5).

3. Device (101; 201; 301; 401) according to Claim 1, wherein the receiver (14) is configured for receiving the electromagnetic radiation (25) reflected by a lens particle (6).

4. Ophthalmic surgical system (100; 200; 300; 400) for phacoemulsification having a control device (101; 201; 301; 401) according to any of the preceding claims and a system component unit (17), which comprises an energy supply (18) for a phaco handpiece (3), and/or an aspiration pump (19), and/or a reflux container (20) for receiving fluid for venting the aspiration line (5).

## Revendications

1. Dispositif de commande (101 ; 201 ; 301 ; 401) destiné à un système de chirurgie ophtalmique (100 ; 200 ; 300 ; 400) pour la phacoémulsification d'une lentille oculaire (2), le système (100 ; 200 ; 300 ; aspirer des particules de d'aspiration (5) (6) conduit d'aspiration (5) s'étendant à l'intérieur d'une aiguille (4) d'une phaco-pièce à main (3) et la phaco-pièce à main (3) pouvant être alimentée en énergie ultrasonique pour briser les particules de lentille en fragments plus petits par vibration longitudinale de l'aiguille,
le dispositif de commande comportant :
un émetteur (10) adapté pour émettre un rayonnement électromagnétique (11),
un dispositif de couplage (26) adapté pour transmettre au moins une partie du rayonnement électromagnétique émis (11) à une extrémité distale (27) du dispositif de couplage (26) et adapté en outre pour injecter l'au moins une partie du rayonnement électromagnétique (11) dans un conduit d'aspiration (5) du système de chirurgie ophtalmique (100 ; 200 ; 300 ; 400), l'extrémité distale (27) du dispositif de couplage (26) étant adaptée pour être disposée à l'extérieur d'un conduit d'aspiration (5),
- un récepteur (14) adapté pour recevoir au moins une partie du rayonnement électromagnétique (13 ; 25),
- une unité de commande (16) avec laquelle une valeur d'un paramètre système de chirurgie ophtalmique (100 ; 200 ; 300 ; 400) peut être commandée en fonction de l'intensité du rayonnement reçu (13 ; 25), le rayonnement électromagnétique (11) ayant une longueur d'onde supérieure à 750 nanomètres, **caractérisé en ce que** le rayonnement électromagnétique est un rayonnement infrarouge pulsé ayant un rayonnement de 0,1 W/cm² maximum

2. Dispositif (101 ; 201 ; 301 ; 401) selon la revendication 1, le récepteur (14) étant adapté pour recevoir le rayonnement électromagnétique (13) sortant du conduit d'aspiration (5).

3. Dispositif (101 ; 201 ; 301 ; 401) selon la revendication 1, le récepteur (14) étant adapté pour recevoir le rayonnement électromagnétique (25) réfléchi par une particule de lentille (6).

4. Système de chirurgie ophtalmique (100 ; 200 ; 300 ; 400) destiné à la phacoémulsification, ledit système comprenant un dispositif de commande (101 ; 201 ; 301 ; 401) selon l'une des revendications précédentes et une unité de composant de système (17) qui comporte une alimentation en énergie (18) destinée à une phaco-pièce à main (3) et/ou une pompe d'aspiration (19) et/ou un récipient à reflux (20) destiné à recevoir le fluide destiné à purger le conduit d'aspiration (5).
